# EUROPEAN PATENT SPECIFICATION

(11) **EP 0 374 162 B1**
(45) Date of publication and mention of the grant of the patent: **29.03.1995**
(21) Application number: 88906615.5
(22) Date of filing: 23.06.1988
(51) Int. Cl.: C12N 15/67, C12N 15/58, C12N 15/70

(54) **BALANCED CONSTITUTIVE INDUCIBLE TRANSCRIPTION SYSTEM**
AUSGEGLICHENES, KONSTITUTIVES, INDUZIERBARES TRANSKRIPTIONSSYSTEM
SYSTEME EQILIBRE DE TRANSCRIPTION CONSTITUTIVE PAR INDUCTION

(30) Priority: 24.06.1987 US 65794
(43) Date of publication of application: 27.06.1990
(73) Proprietor: GENENTECH, INC., South San Francisco California 94080 (US)
(72) Inventor: MILLER, Harvey, I., Pleasant Hill, CA 94523 (US)
(74) Representative: Armitage, Ian Michael
(86) International application number: US8802127
(87) International publication number: WO8810307

(56) References cited:
- EP-A- 0 093 619
- EP-A- 0 136 907
- EP-A- 0 159 779
- EP-A- 0 178 863
- EP-A- 0 236 209
- US-A- 4 643 969
- J. Mol. Biol. (1986) 189, 113-130 (F. WILLIAM STUDIER)
- Proc. Natl. Acad. Sci. USA, vol. 82, pp 1074-1078, February 1985 (STANLEY TABOR),
- Chemical Abstracts, vol. 102, (1985), abstracts no. 144115p, Proc. Natl. Acad. Sci. U.S.A. 1985, 82(4), 1074-8 (Eng).
- Chemical Abstracts, vol. 107 (1987), abstracts no. 128482d, U.S. Pat. Appl. US 905,253,

## Description

### Background

This invention relates to the synthesis of heterologous polypeptides in recombinant host cells. In particular, this relates to the secretion of mature human plasminogen activator proteases from bacterial transformants.

Inducible promoters are well known. Such promoters are characterized by the ability to increase the rate of transcription of genes under their control upon a change in the environment of the cell, typically the concentration of inorganic ions or nutrients, or a change in a physical condition such as the temperature of cultivation. Such promoters include the E. coli trp, E. coli lac and yeast acid phosphatase (Lemontt et al., infra) promoters, inducible by a deficiency in tryptophan, glucose and inorganic phosphate, respectively, or the promoter controlled by the temperature sensitive lambda phage repressor (Harris et al., infra). Inducible promoters have been employed to control the transcription of heterologous genes in recombinant cell culture. In this context, they serve to restrain transcription, and hence translation, of heterologous protein that are known to be toxic to the host cell or which are degraded proteolytically by the host cell. Induction from the promoter at a suitable phase in the recombinant culture therefore minimizes the contact of the host cell with the heterologous polypeptide, and vice versa.

Currently available inducible promoters all exhibit various constitutive levels of transcription, even when conditions are optimized for repression or deactivation of the promoter. For most recombinant systems this is not disadvantageous at the constitutive levels encountered because polypeptide losses to degradation are tolerable, toxicity is insufficient to seriously affect yields of heterologous polypeptide and/or polypeptide shunted into refractile bodies or other processing sinks is readily reactivated or reprocessed.

In an attempt to provide more control over induction of transcription of heterologous genes the T7 RNA polymerase system has been adapted to the synthesis of certain heterologous polypeptides in recombinant host cells.

This system, which has heretofore been extensively described per se, consists of an RNA polymerase that recognizes unique promoters present only in T7 DNA. None of these promoters are known to be transcribed by wild-type E. coli or mammalian RNA polymerases. This transcriptional system containing T7 RNA polymerase and the companion promoters is therefore highly selective, and the selectivity is enhanced by placing the T7 RNA polymerase gene under the control of the lac UV5 inducible promoter recognized by the host cell RNA polymerase (Studier et al., "J. Mol. Biol." 189:113-130 [1986]; Tabor et al.. "Proc. Natl. Acad. Sci. USA" 82:1074-1078 [1985]). Tabor et al., (op. cit.), finding that expression of T7 RNA polymerase was excessive in their system, inserted a transcriptional terminator 5' to the polymerase gene in order to suppress its constitutive expression. This also reduced the inductive capability of the system. A mammalian cell expression system for recombinant cytoplasmic proteins using the T7 RNA polymerase has been described by Fuerst et al., "Proc. Natl. Acad. Sci. USA" 83:8122-8126 (1986). This system as presently constituted, notwithstanding its promise for improving the control of transcription from genes under its aegis, has not proven useful for controlling the expression of certain polypeptides, nor have conventional inducible promoters. In each instance, the constitutive level of transcription from these conventional systems has proven to be incompatible with the host cell and stability of the gene encoding the heterologous protein. The reasons for this incompatibility have been poorly understood. Typically, the art has simply observed that secretion of the desired protein was poor or nonexistent without delving into the mechanisms responsible.

Many heterologous polypeptides have been secreted from microbial hosts. Typically, a chimeric gene is constructed in which a signal sequence is ligated 5' to the codon for the mature amino terminus of the desired polypeptide. This gene is used to transform a microbial host, the host cultured, and the secreted mature polypeptide recovered from the host cell periplasm or, occasionally, the culture medium. Representative general methods for the secretion of heterologous polypeptides from bacteria and yeast are described in Gilbert et al., U.S. 4,338,397 and EP 127,304 or EP 88,632, respectively. It is known to use signal sequences obtained from the host cell ("host-homologous signals"), or signals that are heterologous to the host cell (including the use of variant signals or signals which are native to the heterologous polypeptide). More recently, signals have been directly linked to the mature N-terminus of the heterologous polypeptide, although it also is conventional to link the heterologous polypeptide, together with a portion of its own signal, to the N-terminus of a host-homologous signal. In the alternative, heterologous polypeptide has been linked to the C-terminus of a host-homologous signal containing a portion of the homologous protein normally secreted under the control of the homologous signal.

Previous attempts to provide a microbial host-vector system for the secretion of human plasminogen activator have been unsuccessful, notwithstanding that commercial quantities of tissue type human plasminogen activator (t-PA) are readily secreted from transformants of mammalian cell lines. Bacteria, which do not glycosylate proteins, were first used as hosts for the recombinant synthesis of t-PA. See EP 93,619 and Harris et al. ("Mol. Biol. Med." 3:279-292 [1986]). Harris et al. describe the expression in E. coli of t-PA from a gene encoding N-methionyl t-PA. High levels of recombinant met t-PA did in fact accumulate in the Harris et al. E. coli hosts as insoluble refractile bodies, but little in the way of active enzyme could be recovered, despite extensive efforts to reactivate the enzyme by in vitro processing of the refractile bodies. These authors suggested that expression systems using vectors designed for secretion of proteins in E. coli or yeast may be more successful for making active enzyme under conditions where inclusion bodies are not formed. To date, this prophecy has proven equivocal in the case of yeast and false in the case of bacteria.

Lemontt et al. ("DNA" 4(6):419 [1985]) transformed yeast with t-PA preproteins bearing either the native human t-PA signal or the yeast acid phosphatase signal. Like the E. coli transformants of Harris et al., yeast were found to shunt most of the expressed protein into an insoluble, enzymatically-inactive reservoir (according to Lemontt et al. this occurred as a result of an associative interaction of the t-PA with cell membranes) without the secretion of soluble t-PA into the culture medium. Lemontt et al. did not describe whether the N-termini of the t-PA prepro constructions were properly processed, nor whether the minor proportion of soluble t-PA that was obtained was originally lodged in the periplasm or the cytoplasm.

EP 123,544 reported the secretion of human t-PA by the use of the alpha factor signal. The t-PA was divided equally between the cells (20 »g/l) and the culture medium (20 »g/l).

On the other hand, EP 177,343 observes that little or no human t-PA activity could be obtained from E. coli transformants in which DNA encoding the alkaline phosphatase signal was fused to DNA encoding mature t-PA under the transcriptional control of the alkaline phosphatase promoter. EP 236 209 describes the isolation of human t-PA from E. coli transformants in which the DNA encoding t-PA under the control of P_{L} or Pₜᵣₚ promoter.

The mechanisms responsible for the apparent inability of bacteria to express and secrete human plasminogen activators in commercially significant quantities remain inapparent, although an apparent requirement for glycosylation of the human enzymes has been implicated. Belin et al. ("Eur. J. Biochem." 148:225-232 [1985]) reported the secretion of enzymatically active murine urokinase from E. coli host cells which apparently were transformed with murine preprourokinase, but native murine urokinase is not normally glycosylated. On the other hand, Opdenakker et al. ("Proc. Soc. Exp. Biol. Med." 182:248-257 [1986]) reported significant reductions in the enzymatic activity of secreted t-PA when oocyte transformants were incubated with tunicamycin in order to inhibit glycosylation, the residual enzymatic activity in the tunicamycin-treated oocyte preparations being attributable to partially glycosylated t-PA. Other studies reported in the same work demonstrated reductions in t-PA activity by 15 to 85% when glycosylated t-PA was digested with glycolytic enzymes. Consistent with these findings was an earlier report by Opdenakker et al. ("Eur. J. Biochem." 131:481-487 [1983]) that reticulocyte translation products of the t-PA gene were devoid of enzymatic activity, a phenomenon described by these authors as possibly the result of the inability of reticulocyte preparations to perform post-translational functions (processing and glycosylation). On the other hand, a report by Kagitani et al. ("FEBS" 189(1):145 [1985]) observes enzymatic activity in homogenates of E. coli transformants with DNA encoding des (1-44) "finger domain" deleted variant t-PA. See also Little et al., "Biochemistry" 23(25):6191 (1984) and Rijken et al., "Thromb. and Haem." 54(4):788 (1985, who describe the properties of t-PA having glycosylation reduced by tunicamycin treatment or by enzymatic digestion

If, as suggested by the art, secretion of enzymatically active plasminogen activators that normally are glycosylated will only occur when preplasminogen activators are processed by eukaryotic secretory mechanisms, then it should not be possible to recover commercially significant amounts of secreted enzymatically active plasminogen activators from prokaryotic cell culture.

Accordingly, it is an objective herein to identify problems responsible for the failure of certain host-vector systems to produce desired polypeptides, in particular to stably secrete mature polypeptides from certain preproteins. Realization of this objective will lead to host-vector systems which will successfully and routinely secrete mature heterologous polypeptides.

An additional objective is to devise a method for the secretion of fully functional human plasminogen activators from prokaryotic cell culture.

It is a further objective to provide a method for the secretion of human tissue plasminogen activator (ht-PA) from bacterial cell culture in commercially significant quantities.

It is of great interest to explore the effect of changes in the nucleotide sequences in genes encoding polypeptides, and changes in the amino acid sequences of polypeptides. Typical objectives are to improve yields, alter the characteristics of the polypeptides, study function structure relationships and the like. Conventionally, this is undertaken by introducing a mutation into the nucleotide sequence of the gene of interest, including noncoding (usually 5') and coding domains. The latter may or may not, as desired, be translated into a change in amino acid sequence. In general a single mutation is introduced, the variant gene inserted into an expression vector, and polypeptide expressed, recovered and evaluated. This must be repeated many times before the desired characteristic is achieved since, while most amino acid changes do not radically alter the structure or function of a polypeptide, the effect of any one is unpredictable in most cases. This is a highly laborious and time-consuming process.

The progress of such experiments has been accelerated by the introduction of cassette mutagenesis. In this procedure, random oligonucleotides are synthesized by in vitro methods. Each oligonucleotide has a random target sequence but is also vested with cohesive termini common to all of the oligonucleotides. This allows the insertion of the oligonucleotides into the target gene by first digesting the gene with restriction enzymes that produce termini that are cohesive with the termini of the oligonucleotides, followed by recovering the gene fragment and ligating the oligonucleotides into the gene. These genes are expressed in recombinant culture and the properties of the products evaluated. Many of the recombinants are of no value, but they are readily identified and discarded in the screening process.

The facility of this process breaks down when the polypeptide is one that is preferably made in recombinant mammalian cell culture, whether for ease of recovery or to produce a mammalian polypeptide most like the native molecule. The cost, time and difficulty of mammalian cell culture imposes a burden on random cassette mutagenesis because so many of the cassettes do no result in product having the desired character. A method is needed for easily screening out unproductive mutants, with mammalian cell culture being reserved for confirmation studies. It is an object of this invention to provide such a method.

These and other objects of the invention will be apparent from the specification as a whole.

### Summary

The present invention provides a method for the synthesis in a prokaryotic host cell of a polypeptide which is subject to insolubilization or is toxic in the host cell, comprising transcribing, in a prokaryotic host cell containing an exogenous RNA polymerase DNA integrant, a transforming nucleic acid encoding a preprotein for the polypeptide, in which the presequence effects secretion of the polypeptide, under the control of a promoter recognized by the exogenous RNA polymerase but not recognized by the endogenous host cell RNA polymerase, the expression of exogenous RNA polymerase being under the control of an inducible promoter which, when uninduced, provides a level of expression sufficiently low that the cell culture is stable in that condition over successive generations, whereby said polypeptide is recovered in mature form.

The present invention can be accomplished by a method comprising preparing DNA encoding a polypeptide which is heterologous to a host cell, placing the DNA into an expression vector under the transcriptional control of a balanced constitutive inducible transcriptional control system, transforming a host cell culture with the expression vector, culturing the cell culture to a predetermined cell density, inducing transcription from the DNA encoding said polypeptide, and recovering the polypeptide from the cell culture. The balanced system is obtained by placing the DNA under the transcriptional control of a first inducible promoter system having a first rate of constitutive expression, determining the level of expression of the DNA, and thereafter (a) modifying the rate of constitutive expression of the first promoter system or (b) substituting a second inducible promoter system for the first inducible promoter system, the second promoter system having a rate of constitutive expression which is different from the first promoter system. Typically, if the constitutive level of expression under the control of the first promoter system is excessive one will observe little or no expression of the desired polypeptide, this generally developing over 10-30 generations of host cell replication. In this case the constitutive expression level is reduced by an appropriate manipulation of the first promoter system, e.g. increasing the level of repressor protein in the cell, or by replacing the first promoter system with another having a lower rate of constitutive expression. The promoter is referred to as a "system" in order to include regulatory genes, e.g. those encoding repressors, activators, or dedicated RNA polymerases, that influence transcription from the promoter per se, any one or more of which are manipulated in accordance with this invention.

In the method of this invention an inducible transcriptional control system is used to express a desired host cell-incompatible heterologous polypeptide in recombinant host cell culture wherein the degree of constitutive expression of the polypeptide is balanced against cell incompatibility.

A balanced constitutive inducible system for the transcriptional control of ht-PA in recombinant prokaryotic host cells is provided by 1) modifying DNA 5' to the DNA encoding the T7 RNA polymerase so as to delete a region previously conferring low level constitutive transcriptional activity on the T7 RNA polymerase gene, 2) integrating the gene encoding T7 RNA polymerase, free of other T7 phage promoters, into the genome of the host cell and 3) inverting the T7 promoter in the expression vector so that it does not control the transcription of pBR322 beta-lactamase, thereby suppressing excessive penicillinase expression.

The inventor has observed that the failure to stably excrete heterologous proteins from recombinant host culture frequently is attributable to the use of conventional promoters now found to be inappropriate for use with such proteins. Transformants are stably maintained when the secretion of heterologous preprotein is adjusted to a level compatible with host cell homeostasis, thus making it possible later to obtain elevated levels of secretion upon induction of transcription and translation at a predetermined point in the cultural growth cycle. Otherwise, it has been the inventor's experience that recombinant cells which are sensitive to heterologous preprotein toxicity experience selection pressure against the offending gene over 10-30 generations, resulting for example in retarded cell growth rates, the loss of the gene encoding the protein or, more often, selection for mutants in the protein or its operon that permanently abrogates protein synthesis or results in synthesis of a mutant protein which may be of no commercial utility (but which may be more compatible with the host cell). Specifically, the improved T7 RNA polymerase balanced constitutive transcriptional control system provided herein now facilitates the stable secretion of ht-PA and other polypeptides requiring similar constitutive expression levels. Paradoxically, the secretion of useful quantities of heterologous proteins may be facilitated by selecting a transcriptional control system that does not transcribe the desired gene at maximally elevated levels, but which instead is balanced constitutively with the capabilities of the host cell.

For the first time, water soluble, biologically active, unglycosylated mature t-PA (without an N-terminal methionyl residue) has been obtained from prokaryotes by the use of a balanced constitutive inducible transcription control system. The t-PA nonetheless ordinarily contains the N-linked glycosylation sites present in the native t-PA, i.e. it was not necessary to introduce mutations into these sites. Surprisingly, the t-PA secreted from bacteria is present in commercially significant quantities (yields exceeding about 1 mg/l or 10 ng/OD₅₅₀ unit of culture) and exhibits high specific activity (comparable to that of t-PA from CHO cell transformants). This t-PA is secreted as a water soluble molecule rather than deposited as a refractile body, binds lysine and is fibrin activated, notwithstanding that the bacterial product is unglycosylated and in any case was produced by a method relying solely on microbial processing systems. The secreted t-PA appears to be lodged largely in the inner membrane of the host cell, where it is found in the form of 61 and 57 kilodalton molecules in an approximate ration of 1:20. The 57 kilodalton species is believed to represent mature, processed t-PA while the 61 kilodalton form is probably the t-PA precursor. Smaller molecular weight forms (42 and 35 kilodaltons) are found in the outer membrane. All of these forms of t-PA are water soluble and readily recovered from the cell membranes.

It appears that transcription from pret-PA genes in prokaryotic hosts must be very tightly regulated during the growth phase of pret-PA transformants, more so than has been possible by the use of conventional constitutive or inducible systems such as the heat sensitive λ repressor controlled promoter (Harris et al., op cit) or the alkaline phosphatase promoter and signal (EP 177,343), both of which failed to generate stable host/vector systems that would secrete useful quantities of t-PA. While cytoplasmic t-PA apparently is not toxic, t-PA destined for secretion is toxic to prokaryotes. In the preferred embodiment, stable t-PA secretion is accomplished by placing the t-PA gene under the transcriptional control of the T7 promoter and repressing transcription from the t-PA gene by the use of a host cell containing a novel T7 RNA polymerase DNA integrant which is itself under the transcriptional control of a tightly regulated inducible promoter. When the culture has reached the desired density then, optionally, transcription is induced from the T7 polymerase gene, and then ultimately from the t-PA gene. t-PA is rapidly synthesized and secreted from these transformants at high specific activity and yield.

In summary, therefore, the invention provides a balanced constitutive inducible transtriptional control system particularly for controlling the expression and secretion of a heterologous preprotein such as t-PA. This provides for the first time a method for secreting mature, biologically active, unglycosylated t-PA from bacterial cell culture in commercially useful quantities at high specific activity.

### Brief Description of the Drawings

Fig. 1 depicts the construction of pT7-12, a plasmid providing the phi 10 promoter recognized by T7 polymerase (P_{T7}), and the construction of pT7-12ST2HGH, an intermediate plasmid which supplies the STII signal and phi 10.

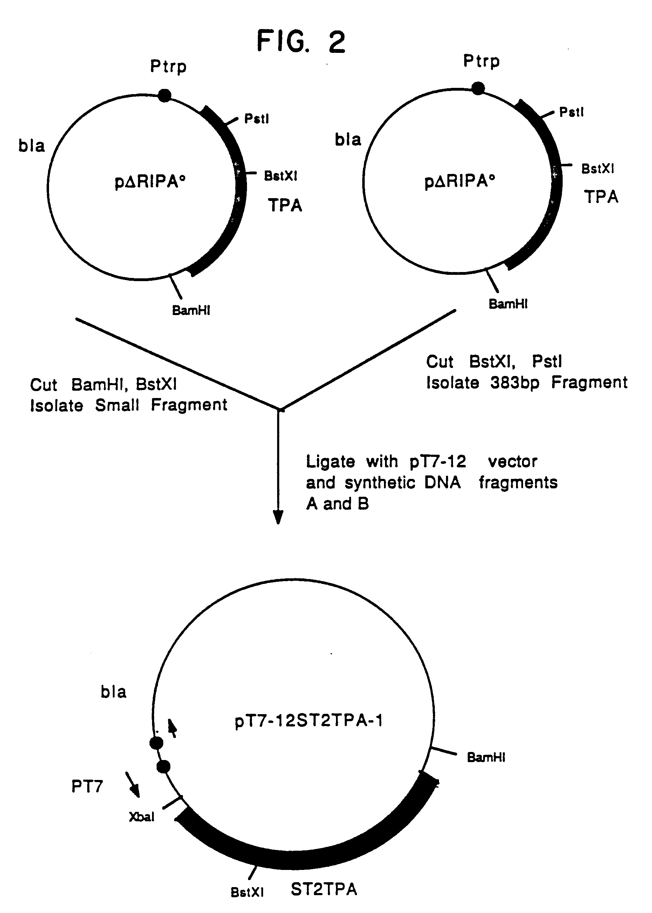

Fig. 2 shows the final steps in the assembly of the pT7-12ST2tPA-1 vector bearing the STII-tPA gene under the transcriptional control of the phi 10 promoter.

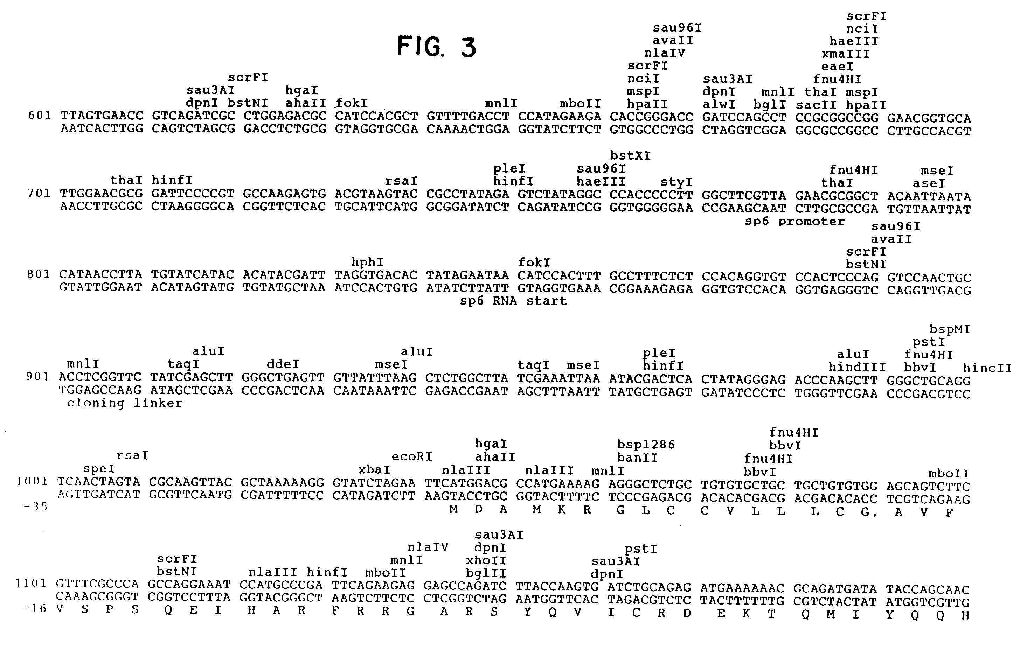

Fig. 3 depicts part of the nucleotide and imputed amino acid sequence of pCT.eik.2 located in the region of the t-PA start codon.

### Detailed Description

It is conventional to place T7 RNA polymerase under the control of the lac UV5 promoter (Studier et al., supra) or the PL promoter (Tabor et al., supra) in order to provide inducibility for T7 RNA polymerase expression. None of these constructions results in the proper constitutive levels of transcriptional control described herein for pre human t-PA. Another construction (Stahl and Zinn "J. Mol. Biol." 148:481-485 [1981]) has now been found to contain a region located 5, to the RNA polymerase gene that acts as a constitutive promoter.

The plasmid employed by Studier et al. (supra) expressed β-lactamase under the control of the T7 promoter, resulting upon induction of the RNA polymerase in an excessive act toxic accumulation or penicillinase in the host culture. This problem was overcome by excising the relevant T7 promoter and gene from the expression plasmid and reinserting it in the inverted orientation so that transcription from the T7 promoter does not lead to polycistronic transcription of the β-lactamase gene.

Finally, the method of Studier et al. (op cit) introduced the RNA polymerase by phage infection, a devastating event from the perspective of the host cell. Even if the prophage was integrated into the host cell genome, the presence of other lambda promoters leads to unsatisfactory elevated constitutive RNA polymerase expression levels. Accordingly, T7 RNA polymerase was integrated into the recipient host genome under the control of an inducible promoter, in the preferred example the lac operon (lac).

The resulting host-vector system, when cultured under conditions of catabolite repression, provides sufficient suppression of RNA polymerase expression that cotransformants with DNA encoding human t-PA grow normally without plasmid instability or host cell toxicity. The constitutive T7 RNA polymerase activity in the balanced constitutive inducible transcription control system herein is less than that which is produced in the Studier et al. (op cit.) host-vector system, but is inducible to much greater levels than can be obtained using the Tabor et al. T7 system.

The T7 system is of the type referred to herein as a "dedicated RNA polymerase transcriptional control system". In such systems the promoter which controls the transcription of a target gene is not recognized, i.e. transcribed, by endogenous host cell RNA polymerase. Besides the T7 system, phage T3 also uses a dedicated RNA polymerase in its expression of T3 proteins. T3 promoters are commercially available (Stratagene). The T3 polymerase add promoters are employed in the same fashion as described herein for the T7 components, i.e., the T3 polymerase is supplied to the host cell under the control of a balanced constitutive inducible promoter preferably as a single copy chromosomal integrant, while a target gene is placed in a high copy number plasmid under the control of a T3 promoter recognized by the T3 polymerase. Similarly, SP6 polymerase and its promoter (Promega Biotec) are employed in the same fashion as T3 and T7.

Other transcriptional control systems which exhibit the proper balanced constitutive expression levels for stable and effective t-PA synthesis will be within the skill of the ordinary artisan. This invention, insofar as it relates to the expression of t-PA, is not limited to dedicated RNA polymerase transcriptional control systems. Other inducible promoters or promoter systems are suitable provided that the level of constitutive expression is balanced for the host cell and t-PA variant concerned. For example, the λ phage CII polypeptide (Fein et al. "Gene" 32:141-150 [1984]) is placed under balanced constitutive inducible transcriptional control. A λ promoter which requires CII polypeptide for transcription by endogenous E. coli RNA polymerase is placed in control of the target gene to be expressed. Then, induction of the CII polypeptide will similarly induce transcription from the target gene.

The balanced constitutive transcriptional control system herein is useful in prokaryotic and eukaryotic host cells including bacterial, fungal (including yeast), mammalian or insect cells. Preferably, the system is used with prokaryotes.

Any polypeptide is capable of being secreted under the control of the balanced constitutive transcriptional control system of this invention, "polypeptide" meaning any polyamide including proteins and low molecular weight peptides having greater than one amino acid residue. As noted above, polypeptides which are subject to insolubilization by host cells are candidates for expression and secretion under the control of the improved system of this invention, as are polypeptides which are toxic to the host cell in which they are expressed. Dedicated RNA polymerase transcriptional control systems are especially useful in cases where a balanced control system requires highly nonconstitutive inducible promoter activity.

Typical polypeptides produced using this method include mammalian or bacterial proteins such as enzymes, hormones, and the like, including particularly mammalian enzymes which are normally glycosylated, e.g. human t-PA, growth factors such as TGF-β, hormones such as activin or prorelaxin, bovine rennin, enkephalinase, and the like.

It will be understood that it is not necessary to use the balanced constitutive control system herein in the procedure for screening nucleotide variants, although it may be useful to do so in cases where it is difficult to achieve secretion of the desired polypeptide from prokaryotes. In the context here, "secretion" refers to the acts of a host cell in processing a preprotein to the mature protein, whether or not the protein is in fact released into the extracellular or periplasmic compartment.

The novel screening procedure of this invention is useful for rapidly and inexpensively identifying variant genes which produce polypeptides in more desirable fashion or having more desirable characteristics. The procedure is most conveniently employed with preproteins in which the signal sequence is recognized both by prokaryotes as well as mammalian cells. The portions of the gene which are varied typically include the 5' noncoding domains about 500 bp upstream from the start codon as well as the protein coding region. The 5' domain may be the sequence native to the gene or it may include a heterologous sequence, e.g. a viral promoter, bacterial Shine-Dalgarno sequence or the like. The coding region generally will include the signal recognized by both hosts. Such signals typically are mammalian signals, or amino acid sequence variants thereof, which are ordinarily associated with the mature polypeptide to be produced. However, the signals also may be heterologous to, i.e., not normally part of, either or both of the mature protein or the intended host cells.

It may be useful to modify the nucleotide or coding sequence of the mammalian signal in order to improve its secretion from prokaryotes. For example, as shown below, the screening method itself can be used to optimize the DNA encoding the signal, i.e., to select for the characteristic of enhanced secretion from prokaryotes and mammalian cells. A t-PA preprotein DNA having a silent mutation within the first about 5 codons of the native signal cDNA is expressed at enhanced levels by prokaryotes, the preferred sequence being ATGGACGCCATGAAA, thus resulting in enhanced levels of secretion.

The plurality of gene variants may range about from 5 to hundreds or thousands of variants. Since the initial prokaryote screen is conducted in cultures wherein each transformant (and hence each variant) is individually evaluated in readily multiplied assays, e.g. as colonies or stabs in petri plates, large numbers can be very efficiently screened; hundreds of colonies can be evaluated simultaneously for chromogenic color formation, activity halos, and the like by simply studying a single plate from which desired colonies can be picked for shuttle vector recovery and transformation of mammalian cells, or protoplast fusion with mammalian hosts. At the least, the microbial culture evaluation permits convenient screening for deleterious mutations, e.g., site directed mutagenesis cassette inserts erroneously containing stop codons or frame shifts. In this case, the characteristic of the polypeptide which is evaluated is activity.

The polypeptide to be screened preferably is a secreted polypeptide from a mammalian source. However, essentially any gene encoding a polypeptide capable of expression in both microbesand higher eukaryotic cells is suitable for mutagenesis and screening herein, including hormones, enzymes and other polypeptides described above.

The structure of the shuttle vector generally contemplates that the microbial expression control sequences will be located between the gene encoding the protein and the mammalian control sequences. The prokaryotic sequences comprise a promoter, preferably the T7 promoter, and a ribosome binding site. The remainder of the vector typically contains elements which are conventional in the art, including a microbial origin of replication, selection genes, mammalian promoters and enhancers, termination sequences and the like.

Once the initial screen is accomplished in microbes the shuttle vector is readily transferred from the microbial cells to higher eukaryotic hosts for transient or stable expression. This is generally followed by evaluation of the expressed protein to confirm that the desired characteristic identified in the microbialscreen also is shared by the eukaryotic cell product, together with any additional evaluation felt necessary at the time. While one will generally use a bacterial screen, other microbes (yeast or fungi) are useful because of their modest culture requirements.

In a preferred embodiment, transcription of preproteins encoding human plasminogen activators is placed under the control of one of the balanced constitutive inducible systems described herein. Plasminogen activators are defined as enzymes which are capable of cleaving plasminogen to produce proteolytically active plasmin. Plasminogen activators include enzymes which bind to fibrin, e.g. tissue plasminogen activator, and those that do not, e.g. urokinase. Also included within the scope of plasminogen activators are engineered amino acid sequence variants not found in nature, e.g., variants in which glycosylation sites are deleted, as well as alleles and animal analogues. The preferred plasminogen activator is full length human t-PA, i.e. t-PA which includes the "finger domain" occurring at the N-terminus of the enzyme. Single or two-chain forms of t-PA fall within the scope of this invention, as do other amino acid sequence variants. Examples of amino acid sequence variants include des Arg₂₇₅ or other variants at residues 270-279 (EP 199,574).

In the preferred embodiments human t-PA is secreted into bacterial periplasm or culture medium from transformants with DNA encoding the native human pret-PA or a fusion of the E. coli STII signal (EP 177,343) with the mature enzyme. However, selection of other suitable signal sequences would be within the skill of the ordinary artisan; the invention here lies in making it possible to readily determine which signals will be most effective by overcoming the incompatibility of pret-PA with prokaryotic hosts.

The balanced constitutive inducible systems typically are constructed by making and cloning all constructions involving the gene for the heterologous protein in the absence of DNA for the promoter system or a key component thereof, e.g. the T3 or T7 RNA polymerase or CII polypeptide gene, thus enabling one to make the constructions without any danger of selection against the protein, for example upon inadvertent derepression of polymerase or CII polypeptide during cloning. Suitable constructions for the desired heterologous protein then are transfected into the desired host cell bearing DNA for the control system, for example the required polymerase or CII genes (or the polymerase or CII genes are transfected into the host cell bearing the heterologous protein DNA). In the preferred embodiment the gene encoding the heterologous polypeptide is placed under the control of the phi 10 promoter (recognized by the T7 polymerase) or a phage T3 promoter (recognized by the T3 polymerase). The tailored T7 RNA polymerase is present as a host cell integrant in E. coli strain K5772 (deposited in the American Type Culture Collection under accession number 53,635). This strain contains the T7 RNA polymerase gene inserted into the chromosomal lacZ operon which is thus inducible by addition of isopropylthiogalactoside (IPTG) to the media. The constitutive level of expression of T7 RNA polymerase is dependent upon the untranslated domain located 5' to the polymerase structural gene. The T7 RNA polymerase gene integrated in E. coli K5772 contains the following sequence (the complementary strand is not shown): 
hinPI
hhaI
mstI
aluI                                    fspI
LacZ-GGGAAGCTAG AGTAAGTAGT TCGCCAGTTA ATAGTTTGCG CAACTTTGTT-
|
HpaI

haeII                                   mnlI
haeI          rsaI                mboII
GCCATCATCT GCGGGTGGCC TGAATAGGTA CGATTTACTA ACTGGAAGAG GCACTAAATG-
polymerase start.
The HpaI site is the first HpaI of the E. coli lacZ.

The balanced constitutive inducible promoter system developed for the secretion of human t-PA from prokaryotes required the introduction of modifications into conventional T7 RNA polymerase vectors, more specifically into the promoter controlling transcription of the T7 RNA polymerase. The lac UV5 promoter of Studier et al. (supra) cannot be sufficiently catabolite repressed (and in fact otherwise has been widely employed because of that characteristic), the Tabor (supra) modified P_{L} promoter could not be sufficiently induced because of the insertion of a terminator, and the Stahl et al. (supra) vector contained an excessively active promoter function of unclear origin located 5' to the T7 RNA polymerase. These inadequacies were overcome by placing the T7 RNA polymerase gene under the control of the wild type lacZ promoter and culturing transformants in the presence of glucose. This wild type lac promoter is constitutively transcribed under catabolite repression at a level which is about 10% of the lac UV5 promoter. The wild type lac promoter is induced about 10 times by catabolite depletion (glucose depletion), and is induced further by adding IPTG to the culture. Without glucose, transcription is excessive, i.e. the lac operon is not sufficiently repressed and t-PA is not secreted. The regulated constitutive level of pret-PA expression achieved with this system has permitted the large scale culture of stable transformants which then can be induced with IPTG or other suitable agent. As a further improvement, lacI^{q} host cells are chosen. These cells overproduce the lac repressor, thus further ensuring that the constitutive expression of pret-PA is sufficiently low to be compatible with the host cell.

The level of constitutive expression from the STII-t-PA preprotein using the lacZ promoted T7 RNA polymerase system described herein was found to be compatible with E. coli K5772 cells. However, it will be appreciated that the optimal expression level of this preprotein, as tolerated by this host, will be determined by the ordinary artisan by appropriate manipulation of preprotein transcription levels. Other host cells and constructions encoding other proteins or preproteins will require balancing the constitutive expression with plasmid stability and host cell toxicity. This is most conveniently accomplished by simply measuring the constitutive mature protein secretion or protein synthesis levels over about from 10 to 50 generations of the transformant. If these constitutive levels are not stable, then the transcription rate of the DNA encoding the preprotein or protein is reduced by further repressing the transcriptional system or by replacing the transcriptional control system entirely with another having an appropriate rate of transcription. On the other hand, if the transformants are stable then the system is modified in the direction of increased constitutive transcription until instability for the selected host-vector system is reached.

The balanced constitutive inducible promoter system is induced at any appropriate point in the host culture cycle, preferably later in the cycle such as the stationary phase or late log phase when cell density is reaching maximum levels. Induction should be rapid rather than progressively phased.

The process of this invention now has facilitated the secretion of full length, mature human t-PA in soluble form which is fully biologically active, i.e. which in the presence of fibrin and plasminogen exhibits proteolytic activity increases of about 10 to 30 times and which is capable of binding to lysine Sepharose. The human t-PA of this invention is unglycosylated yet is soluble and exhibits high specific activity. In addition, in the preferred embodiment it contains the finger region located between residues 1 and 44, and ordinarily the novel t-PA of this invention contains the complete amino acid sequence of native human t-PA. However, it will be appreciated that amino acid sequence variants, especially substitutional variants, also are included within the scope of t-PA as produced by the method herein.

A portion of the t-PA is secreted into prokaryotic host periplasm at levels of specific activity exceeding about 2 S-2251 units at A₄₀₅/min/»g of t-PA protein as determined by enzyme linked immunoabsorbent assay (ELISA). Specific activity typically ranges about from 2 to 10 S-2251 units. This degree of specific activity is comparable to that of recombinant t-PA secreted from mammalian cell culture and far exceeds the activity of non-secreted met-PA obtained from the cytoplasm of prokaryotic transformants. It was surprising and unexpected that unglycosylated human t-PA would be soluble in aqueous media (10 mM Tris pH 8 and 0.5 m arginine) at concentrations exceeding 50 »g/ml and yet retain the salient enzymological functions of the glycosylated form of the molecule.

The processed (mature) t-PA herein is recovered from the periplasmic space by conventional methods, e.g. isotonic or cold shock or from cell membranes by extraction into aqueous solutions of detergents. Measures desirably are taken during recovery of the t-PA in order to protect it from hydrolysis by endogenous host proteases, e.g. by flooding the recovery solutions with an innocuous substrate peptide to swamp out adventitious proteolytic enzymes and by conducting the recovery as rapidly as possible to avoid exposing the t-PA to cytoplasmic proteases. Alternatively, host cells are selected for an absence of interfering proteases.

In order to simplify the Examples, certain frequently occurring methods will be referenced by shorthand phrases.

"Plasmids" are designated by a low case p preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

"Digestion" of DNA refers to catalytic cleavage of the DNA with an enzyme that acts only at certain locations in the DNA. Such enzymes are called restriction enzymes, and the sites for which each is specific is called a restriction site. The various restriction enzymes used herein are commercially available and their reaction conditions, cofactors and other requirements as established by the enzyme suppliers were used. Restriction enzymes commonly are designated by abbreviations composed of a capital letter followed by other letters representing the microorganism from which each restriction enzyme originally was obtained and then a number designating the particular enzyme. In general, about 1 »g of plasmid or DNA fragment is used with about 2 units of enzyme in about 20 »l of buffer solution. Appropriate buffers and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol. Digestion with a restriction enzyme infrequently is followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional (T. Maniatis et al., 1982, Molecular Cloning pp. 133-134).

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing tie desired fragment, and separation of the gel from DNA. This procedure is known generally. For example, see R. Lawn et al., 1981, "Nucleic Acids Res." 9:6103-6114, and D. Goeddel et al., 1980, "Nucleic Acids Res.: 8:4057.

"Transformation" or "transfection" means introducing DNA into an organism so that the DNA is replicable, either as an extrachromosomal element or chromosomal integrant. Unless otherwise provided, the method used herein for transformation of E. coli is the CaCl₂ method of Mandel et al., 1970, "J. Mol. Biol." 53: 154.

"Ligation" refers to the process of forming phosphodiester bonds between two double stranded nucleic acid fragments (T. Maniatis et al., Id., p. 146). Unless otherwise provided, ligation may be accomplished using known buffers and conditions with 10 units of T4 DNA ligase ("ligase") per 0.5 »g of approximately equimolar amounts of the DNA fragments to be ligated.

All literature citations herein are expressly incorporated by reference.

### EXAMPLE 1

### Construction of pT7-12

Plasmid pT7-2 was obtained from United States Biochemical Corporation. The plasmid DNA was cleaved with PvuI and religated. The DNA was used to transform competent bacteria and clones screened for inversion of the PvuI fragment. One such inverted clone was called pT7-12. The purpose of this construction was to prevent high-level expression of the beta-lactamase gene, which otherwise would be transcribed under the control of the phi 10 promoter.

### Construction of pT7-12ST2HGH

Plasmid pT7-12 was digested with HinCII and BamHI and the large vector fragment isolated. Plasmid ptrpST2HGH (EP 177,343) was digested with HpaI and BamHI and the small hGH coding fragment isolated. The two fragments were ligated to produce pT7-12ST2HGH.

### Construction of pT7-12ST2t-PA

Plasmid pT7-12ST2HGH was digested with XbaI and BamHI and the large vector fragment isolated. Plasmid pΔRIPA° (EP 93,619, containing the E. coli trp promoter/operator and the human t-PA gene) was digested with BstXI and BamHI and the small fragment (the t-PA gene) isolated. The same plasmid was also cleaved with BstXI and PstI and the 383bp fragment isolated. These three fragments were ligated with two double-stranded synthetic DNA fragments A (the N-terminus of the STII signal) and B (encoding the C-terminus of the STII signal fused to the N-terminal seryl residue of t-PA) to yield pT7ST2t-PA-1.

XbaI MluI
CTAGAATTATGAAAAAGAATATCGCATTTCTTCTTGCATCGATGTTCGTTTTTTCTATTGCTACAAA
TTAATACTTTTTCTTATAGCGTAAAGAAGAACGTAGCTACAAGCAAAAAAGATAACGATGTTTGCGCp
MetLysLysAsnIleAlaPheLeuLeuAlaSerMetPheValPheSerIleAlaThrAsn

Fragment A

MluI PstI
pCGCGTATGCATCTTACCAAGTGATCTGCA
ATACGTAGAATGGTTCACTAG
AlaTyrAlaSerTyrGln[?]alIleCys

Fragment B

### Introduction of pT-12ST2t-PA into an Expression Host

K5772 bacteria were made competent and transformed with pT7-12ST2t-PA-1 by selecting for resistance to ampicillin. The transformed bacteria were maintained on media containing glucose to lower expression by catabolite repression of the lac operon, which controls the level of T7 RNA polymerase.

### Expression and Secretion of ST2t-PA in E.coli

K5772 containing pT7-12ST2t-PA-1 (K5776) was grown at 37°C to saturation in 1 litre of M9-glucose -casamino acid media with ampicillin, the cell pellet collected by centrifugation and stored at -20°C. The presence of glucose ensures catabolite repression of the lac promoter controlling T7 RNA polymerase transcription. Appreciable levels of ST2t-PA were produced even under conditions of constitutive repression by glucose; addition of IPTG to the culture resulted in the expression of greater quantities of t-PA as adjudged by gel electrophoresis of cell extracts.

### Extraction of t-PA from the Host Cells

The cell pellet was thawed and resuspended in an aqueous solution containing 0.1 M Tris-HCl pH8, 0.05 M EDTA, and 0.5 M sucrose at a cell density of 250 OD units/ml at 0°C. The cells were stirred for 10 min. The cells were centrifuged and the supernatant discarded. A. To prepare periplasmic t-PA, the pellet was resuspended in 10 mM Tris-HCl pH8 at 250 OD units/ml. Once the pellet was resuspended, solid arginine-HCl was added to produce a concentration of 0.5 M (105 g/L). The suspension was stirred for 2 hr. at 0°C after which the cells were removed by successive centrifugation at 12,000 x g for 20 min. B. To extract t-PA from the membrane, the cells were extracted in 1% Triton X-100, 0.1M Tris-HCl pH 8, 0.05 M EDTA at 37°C for 1 hr., after which the cells (which do not lyse after this incubation) are removed by centrifugation. The soluble t-PA is located in the Triton extract, to which arginine - HCl can be added as provided in the periplasmic extraction and the detergent removed by dialysis.

### Purification of the Extracts

An antibody-sepharose column of rabbit polyclonal anti-CHO-tPA antibody was equilibrated with 0.1M HEPES pH7.5, 0.5M Arginine. Either of the E. coli extracts, in the same buffer, were loaded on the column. The column was washed with 50mM Tris pH7.5, 1 M NaCl until no further protein (as detected by an in-line adsorbance monitor) was in the wash. The t-PA then was eluted with 0.1 M Acetic acid, 0.15 M NaCl pH3, the peak collected and the t-PA solution titrated to pH 7 with 2 N KOH.

### Assays for t-PA

### (a) Specific Activity and Fibrin Stimulation

The purified periplasmic extract was assayed for specific activity by S-2251 with fibrinogen to determine activated plasmin activity and by ELISA for t-PA protein to determine the amount of t-PA responsible for the activity. Assays without fibrinogen were used to determine fibrin stimulation. The reaction mixture contained 30 »l of a plasminogen solution (2.1 mg/ml) with or without fibrinogen (20 »l of a 20 mg/ml solution) in a final total volume of 150 »l of 0.05 M Tris-HCl, pH 7.4, 0.12 M sodium chloride, and 0.01% Tween 80. A total of 10 »l of the sample to be tested was added, containing 2-5 ng of enzyme for reactions with fibrinogen and between 0.05 and 2 »g for reactions without fibrinogen. The reaction was allowed to proceed for 10 min at 37°C. The reaction mixture was diluted with 0.35 ml of 0.86 mM H-D-valyl-L-leucyl-L-lysine-p-nitroanilide (S-2251) dissolved in 36 mM Tris, 0.086 M NaCl, and 0.007% Tween 80. Plasmin cleavage of the S-2251 was allowed to proceed for 5 min, and the reaction was terminated by the addition of 25 »l of glacial acetic acid. The extent of plasmin formation was determined by the absorbance of the sample at 405 nm. A "unit" of S-2251 is defined as 1 A₄₀₅/min/»g t-PA protein.

The ELISA for t-PA was conducted as follows:
The wells of plastic microtiter plates were coated with goat anti-CHO-tPA polyclonal antibody by adding 100 microliters of antibody in coating buffer (0.05 M sodium carbonate pH9.6) to each well and incubating at 4°C for 12 hrs. "CHO-tPA" is essentially homogeneous human t-PA secreted by and recovered from the culture media of recombinant Chinese Hamster Ovary cells transformed with the gene for human t-PA. The wells were washed three times with 200 »l wash buffer (0.01 M sodium phosphate pH 7.4(PBS), 0.05% Polysorbate 80), 200 »l of assay diluent are added to each well and incubated for one hr. at room temperature with agitation. The assay diluent was PBS, 0.5% Bovine Serum Albumin, 0.01% Polysorbate 80, 0.01% Thimersol. The wells were washed again with wash buffer. CHO-tPA standards, untransformed E. coli periplasmic extract and samples of periplasmic t-PA extracts were added in 100 »l of assay diluent to the wells and incubated at room temperature with agitation for 2 hr, after which the wells were washed with wash buffer. 100 »l of goat polyclonal anti-tPA-HRP conjugate in assay diluent were added to each well and incubated at room temperature for 1 hr with agitation, followed by another wash. 100 »l of HRP substrate was added to each well and incubated for 20 min in the dark at room temperature. HRP substrate is 0.04% o-phenylenediamine, 0.1M Citric Acid, 0.2M sodium phosphate pH 5, 0.12% Hydrogen Peroxide. 100 »l of 4.5 N sulfuric acid was added to each well and the adsorbances were read at 492 nm. The sample values are compared with the standard curve.

The secreted E. coli t-PA appears to increase in titer in this assay as the dilution is increased. This is attributed to the presence of water soluble multimers of the t-PA at high concentrations which are detected by the ELISA assay as monomers. Therefore, the samples must be diluted until the point is reached where no further increase in titer is seen with increasing dilution or this must be extrapolated from available data. The t-PA concentration determined in this manner is used in the specific activity determination.

A »g of t-PA protein is defined as the amount of t-PA giving the same A₄₉₂ as the 1 »g standard.

The purified extract was found to contain 3.9 S-2251 A₄₀₅ units/min./»g of t-PA protein. The yield of t-PA was 1 mg/l.

### (b) Lysine Binding

A column of lysine-sepharose was equilibrated with 50 mM sodium phosphate buffer pH 7.5. The purified extract was dialyzed into 25 mM Tris pH8/0.5M NaCl and loaded on the column. The column then was washed with 40 mM Tris pH8/0.5M NaCl and the bound protein eluted with 50 mM sodium phosphate buffer pH7.5/0.2M arginine. Column fractions were assayed for t-PA activity by fibrin plate assay. The results of this study demonstrated that >90% of the total loaded t-PA activity bound to the lysine column.

### EXAMPLE 2

### Construction of t-PA in E. coli - Mammalian Cell Shuttle Secretion Vector

This example describes the construction of a single plasmid vector capable of secreting t-PA in E. coli as well as mammalian cells. Since translation in mammalian cells is mostly insensitive to the 5' untranslated region (as long as a competing AUG is absent), the crucial part was finding a variant of the mammalian cell-compatible signal sequence which also is capable of expression and secretion by E. coli. This was accomplished by substituting all possible nucleotide sequences in the first five codons of the t-PA cDNA (avoiding amino acid changes) and ligating these variants to a strong E. coli ribosome binding site. The ability to secrete active t-PA was tested by stabbing E. coli transformed with these plasmids into fibrin plates (Alkjaersig et al., "J. Clin. Invest." 38:1086-1095 [1959]). Colonies secreting t-PA show a halo of lysis in this assay.

### Construction of pT7-3ST2t-PA

pAPSTIIhGH (EP 177,343) is digested with NdeI and AvaI, filled, ligated and pAPSTIIhGH ΔN-A recovered in which the sequence between the NdeI and AvaI sites is deleted.

pAPSTIIhGHΔN-A is digested with HindIII, filled, digested with BamHI and the vector fragment (I) recovered.

One aliquot of pT7-12ST2t-PA-1 was simultaneously digested with XbaI and BamHI, and another aliquot was digested with XbaI and PvuII. The 92 bp PvuII-XbaI pT7-containing fragment (II) and the approx. 2000 bp XbaI-BamHI fragment containing the t-PA gene (III) were recovered from the digest.

Fragments I, II and III were ligated and pT7-3ST2t-PA was recovered.

### Construction of pT7-3ST2t-PA ΔbsmI

Plasmid pT7-3ST2t-PA was cut with bsmI and the large vector fragment isolated. The ends were blunted with Klenow enzyme and ligated to yield pT7-3ST2t-PA ΔbsmI which contains a single XbaI and Bgl2 site.

### Mutagenesis of cDNA Sequence

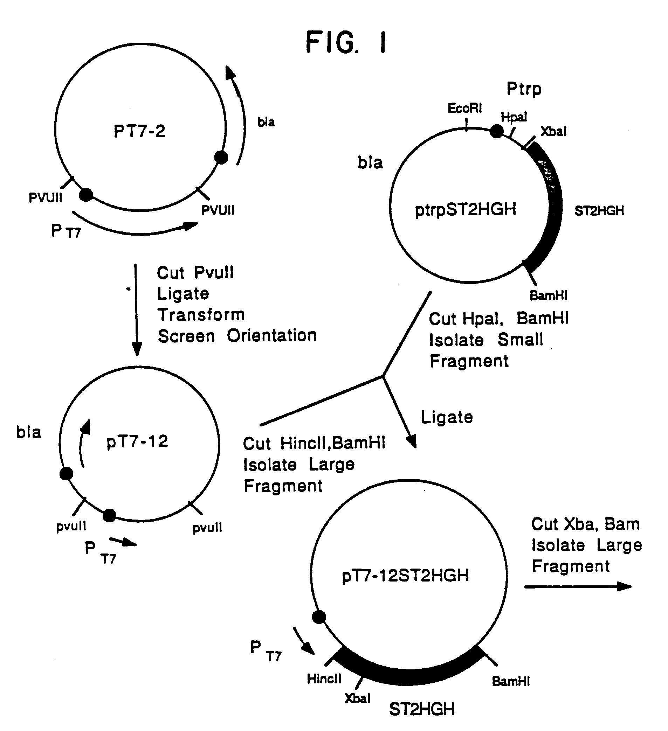

Plasmid pT7-3ST2t-PA ΔbsmI was cut with XbaI and Bgl2 and the large vector fragment was isolated (Fig. 1). An 85 bp fragment of preprot-PA extending from the Ban2 site at G⁻²⁹ to the Bgl2 site at R⁻¹ is isolated from the preprot-PA gene. Synthetic DNA was synthesized 
XbaI          met asp   ala met lys   arg gly   banII
5' CTA-GAA-TTC-ATG-GAT/C-GCX-ATG-AAA/G-AGA-GGG-CT
          TT-AAG-TAC-CTA/G-CGX-TAC-TTT/C-TCT-C 5'
which coded for all 16 possible conservative nucleotide changes in the first 5 codons of t-PA and which has XbaI and Ban2 compatible ends. The vector, 85 mer and synthetic DNA were ligated together and transformed into E. coli strain K5772. The colonies were stabbed into fibrin plates and the DNA analyzed for the proper restriction map. One plasmid shown to have the proper structure and which gave a large halo on fibrin plates was selected for DNA sequencing. DNA sequencing showed that this clone, pT7-3preprot-PA-3 contained three mutations in the first five codons of the cDNA coding sequence: 
ATG GAC GCC ATG AAA
M D A M K
As shown, none of the mutations altered the amino acid sequence of preprot-PA.

### Construction of pRK5

The starting plasmid was pCIS2.8c28D (described in EP 260,148). The base numbers in paragraphs 1 through 6 refer to pCIS2.8c28D with base one of the first T of the EcoRI site preceding the CMV promoter. The cytomegalovirus early promoter and intron and the SV40 origin and polyA signal were placed on separate plasmids.
1. The cytomegalovirus early promoter was cloned as an EcoRI fragment from pCIS2.8c28D (9999-1201) into the EcoRI site of pUC118 (Yanish-Perron et al. Gene 33:103 [1985]). Twelve colonies were picked and screened for the orientation in which single stranded DNA made from pUC118 would allow for sequencing from the EcoRI site at 1201 to the EcoRI site at 9999. This clone was named pCMVE/P.
2. Single stranded DNA was made from pCMVE/P in order to insert an SP6 (Green, M.R. et al., Cell 32:681-694 [1983]) promoter by site-directed mutagenesis. A synthetic 110mer which contained the SP6 promoter (See Nucleic Acids Res. 12:7041 [1984] figure 1; sequences from -69 to +5 of SP6 promoter were used along with 18bp fragments on either end of the oligomer corresponding to the CMVE/P sequences. Mutagenesis was done by standard techniques and screened using a labeled 110 mer at high and low stringency. Six potential clones were picked and sequenced. A positive was identified and labelled pCMVE/PSP6.
3. The SP6 promoter was checked and shown to be active, for example, by adding SP6 RNA polymerase and checking for RNA of the appropriate size.
4. A ClaI-NotI-SmaI adapter was made to be inserted from the ClaI site (912) to the SmaI site of pUC118 in pCMVE/P (step 1) and pCMVE/PSP6 (step 2). This adapter was ligated into the ClaI-SmaI site of pUC118 and screened for the correct clones. The linker was sequenced in both and clones were labelled pCMVE/PSP6-L and pCMVE/P-L.
5. pCMVE/PSP6-L was cut with SmaI (at linker/pUC118 junction) and HindIII (in pUC118). A HpaI (5573) to HindIII (6136) fragment from pSVORAAΔRI 11, described below, was inserted into SmaI-HindIII of pCMVE/PSP6-L. This ligation was screened and a clone was isolated and named pCMVE/PSP6-L-SVORAAΔRI.
   a) The SV40 origin and polyA signal was isolated as XmnI (5475) - HindIII (6136) fragment from pCIS2.8c28D and cloned into the HindIII to SmaI sites of pUC119. This was named pSVORAA.
   b) The EcoRI site at 5716 was removed by partial digest with EcoRI and filling in with Klenow. The colonies obtained from self-ligation after fill-in were screened and the correct clone was isolated and named pSVORAAΔRI 11. The deleted EcoRI site was checked by sequencing and shown to be correct.
   c) The HpaI (5573) to HindIII (6136) fragment of pSVORAAΔRI 11 was isolated and inserted into pCMVE/PSP6-L (see 4 above).
6. pCMVE/PSP6-L-SVOrAAΔRI (step 5) was cut with EcoRI at 9999, blunted and self-ligated. A clone without an EcoRI site was identified and named pRK.
7. pRK was cut with SmaI and BamHI. This was filled in with Klenow and religated. The colonies were screened. A positive was identified and named pRKΔBam/Sma 3.
8. The HindIII site was converted to a HpaI site using a converter. (A converter is a piece of DNA used to change one restriction site to another. In this case one end would be complimentary to a HindIII sticky end and at the other end have a recognition site for HpaI.) A positive was identified and named pRKΔBam/Sma, HIII-HpaI 1.
9. pRKΔBam/Sma, HIII-HpaI 1 was cut with PstI and NotI and a RI-HIII linker and HIII-RI linker were ligated in. Clones for each linker were found. However, it was also determined that too many of the HpaI converters had gone in (two or more converters generate a PvuII site). Therefore, these clones had to be cut with HpaI and self-ligated.
10. RI-HIII clone 3 and HIII-RI clone 5 were cut with HpaI, diluted, and self-ligated. Positives were identified. The RI-HIII clone was named pRK5.

### Construction of pRK.eik.2

The EIK variant of human t-PA was selected as the gene for use in the screening method herein. This variant is described in EP 199,574. It is characterized by substitutional mutations that eliminate a proteolysis site.

Any plasmid bearing this variant, e.g. pPADHFR-6 2C9 from EP 199,574 is digested with BstEII to completion and partially digested with HgaI. The t-PA-containing 1800 bp fragment (I) is recovered. The HgaI and BstEII sites are respectively located near the 5' and 3' ends of the EIK t-PA coding sequence (the BstEII site being about 40 bp 5' to the stop codon). A synthetic oligonucleotide is prepared (fragment II) having the sequence 
BstEII
5' GTTACCAACTACCTAGACTGGATTCGTGACAACATGCGACCGTGA 3'
GTTGATGGATGAGACCTAAGCACTGTTGTACGCTGGCACTGC 5'
This is the 3' end of the t-PA gene. Finally, a second synthetic oligonucleotide (fragment III) is prepared: 
HgaI
5' CGAGCTTGGGCTGCAGGCCGTGAATTTAAGGACGCTGTGA 3'
TCGAACCCGACGTCCGGCACTTAAATTCCTGCGACACTTCGTT 5'
The two synthetic DNAs (fragments II and III) are ligated to the 1800 bp HgaI-BstEII fragment (I) to produce (IV).

pRK5 is digested with ClaI and AccI (vector fragment V). The t-PA gene (IV) is ligated to the pRK vector (V) and pCT.eik.2, containing the t-PA gene in the proper orientation, is recovered.

### Construction of pCT.eik.2

Plasmid pRK.eik.2 was cleaved with speI and bgl2 and the large vector fragment isolated. The same plasmid was cleaved with TaqI, the ends blunted with Klenow enzyme, then cut with speI and the 885 bp fragment isolated. Plasmid pT73preprot-PA3 was cut with DdeI and the ends blunted with Klenow enzyme, then cut with bgl2 and the 225 bp fragment isolated. The vector and two fragments were ligated together to generate pCT.eik.2. The plasmid comprises the CMV promoter/enhancer region, the immunoglobulin splice acceptor, the Sp6 promoter and RNA start, the T7 promoter, the trpE leader ribosome binding site, the altered preprot-PA gene, and the SV40 polyA site. This construction is capable of transient expression, i.e., it is not necessary to use a selection gene to obtain secretion from mammalian transformants. However, the same or other constructions are transfected into mammalian cells together with a selection gene (e.g. DHFR or a neomycin resistance gene) and exposed to an agent for identifying cells having taken up and amplified DNA, e.g. methotrexate or neomycin, in accord with methods known per se in the art.

It also is within this invention to employ other mammalian expression control elements, e.g. other viral (SV40) promoters and enhancers, in place of the CMV promoter/enhancer and the splice acceptor. Such elements are widely known and available in the art.

### Expression of t-PA from pCT.eik.2 in E. coli and Mammalian Cells

Plasmid pCT.eik.2 was transformed into strain K5772 and the colonies were stabbed into fibrin plates. The plates were incubated overnight at 37°C for 24 hrs. A halo of lysis surrounding the stabbed colonies indicated t-PA activity. In addition, DNA of pCT.eik.2 was transfected into human embryonic kidney (293) cells. After 48 hr. incubation, t-PA in the supernatant was assayed for t-PA by ELISA assay. Plasmid pCT.eik.2 produced at least as much t-PA as a wild-type control plasmid.

### Uses of pCT.eik.2 Secretion Shuttle Vector

The primary use of the secretion shuttle vector described herein is for screening mutants of t-PA, constructed by in vitro mutagenesis, for various characteristics such as protease activity, fibrin binding and sensitivity to specific and nonspecific protease inhibitors prior to transfecting mammalian cells with mutant plasmid DNA. The conventional alternative, that of screening random site-directed mutations by transfection into mammalian cells and recovering cell supernatants for individual study, is prohibitively time and labor consuming. However, by prescreening mutants in prokaryotes such as non-expressing clones can be eliminated and clones with new character are readily detected with little investment in time and labor.

In a typical embodiment, a plurality of cassettes are prepared by in vitro synthesis that contain random mutations in a target region of the t-PA molecule, e.g. the growth hormone domain, finger region, kringles or the like. Generally, each of these cassettes have 5' and 3' terminal cohesive termini capable of ligation to restriction sites flanking the target region of t-PA. The shuttle secretion plasmid described herein is digested with the relevant restriction enzyme(s), the vector fragment recovered and ligated to a random member of the cassette mixture. The resulting vector population contains DNA encoding nucleotide and, in most cases, amino acid sequence variants of the t-PA gene, including deletion variants where the cohesive termini permit religation without cassette insertion. The nucleotide variants may contain stop codons or may result in a frame shift, both of which generally result in a highly modified t-PA sequence. Other nucleotide variants will encode substitutional, insertional or deletional modifications in the amino acid sequence. Since it is difficult to predict how any one variant will perform with respect to a desired. quality, e.g. enzymatic activity, fibrin binding, resistance to plasma inhibtors and the like, the advantage of the method described herein is that such variants are preliminarily screened in a microbial secretion system using a vector that is conveniently transferred to a mammalian secretion system for confirmation of the results seen in the screen of the microbial secretory product. This transfer into a mammalian system is readily accomplished by recovering, by methods known per se, the vector from microbial colonies secreting t-PA which demonstrates the desired character. Alternatively, and more conveniently, spheroplasts of bacteria are prepared and fused with the desired mammalian cell hosts using methods known per se. This avoids the need to isolate plasmids from the microbial hosts.

## Claims

1. A method for the synthesis in a prokaryotic host cell of a polypeptide which is subject to insolubilization or is toxic in the host cell, comprising transcribing, in a prokaryotic host cell containing an exogenous RNA polymerase DNA integrant, a transforming nucleic acid encoding a preprotein for the polypeptide, in which the pre-sequence effects secretion of the polypeptide, under the control of a promoter recognized by the exogenous RNA polymerase but not recognized by endogenous host cell RNA polymerase, the expression of exogenous RNA polymerase being under the control of an inducible promoter which, when uninduced, provides a level of expression sufficiently low that the cell culture is stable in that condition over successive generations, whereby said polypeptide is recovered in mature form.

2. The method of claim 1 wherein the RNA polymerase is T7 RNA polymerase.

3. The method of claim 2 wherein the T7 RNA polymerase is under the control of an inducible promoter and the nucleic acid encoding the polypeptide is under the transcriptional control of a T7 promoter recognized by T7 RNA polymerase.

4. The method of any one of claims 1, 2 and 3 wherein said polypeptide is a human plasminogen activator.

5. The method of claim 4 wherein the human plasminogen activator is tissue-type plasminogen activator.

6. The method of any one of the preceding claims wherein the polypeptide is recovered in mature form from the host cell periplasm, membrane or the cell cuture medium.

7. A method for the secretion from prokaryotic host cells of biologically active human t-PA which is subject to insolubilisation or is toxic in the host cell, the host cells containing an exogenous RNA polymerase DNA integrant and transformed with nucleic acid encoding a preprotein of human t-PA in which the presequence effects the secretion of the t-PA in the host cell, said nucleic acid being under the transcriptional control of a promoter recognized by the exogenous RNA polymerase but not recognized by endogenous host cell RNA polymerase, the expression of exogenous RNA polymerase being under the control of an inducible promoter which, when uninduced, provides a level of expression sufficiently low that the cell culture is stable in that condition over successive generations, the method comprising culturing the transformants, inducing transcription and recovering mature human t-PA from the cell periplasm, membrane or the cell culture medium.

## Patentansprüche

1. Verfahren zur Synthese eines Polypeptids in einer prokaryotischen Wirtszeile, das in der Wirtszelle insolubilisiert wird oder toxisch ist, umfassend, in einer prokaryotischen Wirtszelle, die einen exogenen RNA-Polymerase-DNA-Integrator enthält, das Transkribieren einer transformierenden Nukleinsäure, die für ein Präprotein für das Polypeptid kodiert, in dem die Prä-Sequenz die Sekretion des Polypeptids bewirkt, unter der Steuerung durch einen Promotor, der von der exogenen RNA-Polymerase, jedoch nicht von endogener Wirtszellen-RNA-Polymerase erkannt wird, wobei die Expression von exogener RNA-Polymerase unter der Steuerung eines induzierbaren Promotors erfolgt, der, falls nicht induziert, eine ausreichend niedrige Expressionsmenge liefert, so daß die Zellkultur in diesem Zustand über aufeinanderfolgende Generationen stabil ist, wodurch das Polypeptid in reifer Form gewonnen wird.

2. Verfahren nach Anspruch 1, worin die RNA-Polymerase T7-RNA-Polymerase ist.

3. Verfahren nach Anspruch 2, worin die T7-RNA-Polymerase unter der Steuerung eines induzierbaren Promotors und die für das Polypeptid kodierende Nukleinsäure unter transkriptionaler Steuerung eines T7-Promotors steht, der von T7-RNA-Polymerase erkannt wird.

4. Verfahren nach irgendeinem der Ansprüche 1, 2 und 3, worin das Polypeptid ein menschlicher Plasminogenaktivator ist.

5. Verfahren nach Anspruch 4, worin der menschliche Plasminogenaktivator ein Plasminogenaktivator vom Gewebetyp ist.

6. Verfahren nach irgendeinem der vorangegangenen Ansprüche, worin das Polypeptid in reifer Form aus Wirtszellen-Periplasma, -Membran oder dem Zellkultur-Medium gewonnen wird.

7. Verfahren zur Sekretion von biologisch wirksamem menschlichem t-PA aus prokaryotischen Wirtszellen, der in der Wirtszelle insolubilisiert wird oder toxisch ist, wobei die Wirtszellen einen exogenen RNA-Polymerase-DNA-Integranten enthalten und mit Nukleinsäure transformiert werden, die für ein Präprotein von menschlichem t-PA kodiert, bei dem die Präsequenz die Sekretion des t-PAs in der Wirtzelle bewirkt, wobei die Nukleinsäure unter der transkriptionalen Steuerung durch einen Promotor steht, der von der exogenen RNA-Polymerase, jedoch nicht von endogener Wirtszellen-RNA-Polymerase erkannt wird, wobei die Expression von exogener RNA-Polymerase unter der Steuerung eines induzierbaren Promotors erfolgt, der, falls nicht induziert, eine ausreichend niedrige Expressionsmenge liefert, so daß die Zellkultur in diesem Zustand über aufeinanderfolgende Generationen stabil ist, wobei das Verfahren das Kultivieren der Transformanten, das Induzieren von Transkription und das Gewinnen von reifem menschlichem t-PA aus Zellperiplasma, -membran oder dem Zellkultur-Medium umfaßt.

## Revendications

1. Procédé pour la synthèse, dans une cellule-hôte procaryotique, d'un polypeptide qui subit une insolubilisation ou qui est toxique dans la cellule-hôte, comprenant la transcription, dans une cellule-hôte procaryotique contenant un produit d'intégration d'ADN codant pour une ARN-polymérase exogène, d'un acide nucléique à effet transformant codant pour une préprotéine pour le polypeptide, dans lequel la préséquence effectue la sécrétion du polypeptide, sous le contrôle d'un promoteur reconnu par l'ARN-polymérase exogène mais non reconnu par l'ARN-polymérase endogène de la cellule-hôte, l'expression de l'ARN-polymérase exogène étant sous le contrôle d'un promoteur inductible qui, à l'état non induit, engendre un degré d'expression suffisamment bas pour que la culture cellulaire soit stable dans cette condition au cours de générations successives, ce qui permet de recueillir ledit polypeptide sous forme mature.

2. Procédé suivant la revendication 1, dans lequel l'ARN-polymérase est l'ARN-polymérase de T7.

3. Procédé suivant la revendication 2, dans lequel l'ARN-polymérase de T7 est sous le contrôle d'un promoteur inductible et l'acide nucléique codant pour le polypeptide est sous le contrôle transcriptionnel d'un promoteur de T7 reconnu par l'ARN-polymérase de T7.

4. Procédé suivant l'une quelconque des revendications 1, 2 et 3, dans lequel le polypeptide est un activateur du plasminogène humain.

5. Procédé suivant la revendication 4, dans lequel l'activateur du plasminogène humain est l'activateur du plasminogène de type tissulaire.

6. Procédé suivant l'une quelconque des revendications précédentes, dans lequel le polypeptide est recueilli sous forme mature à partir du cytoplasme de la cellule-hôte, de la membrane de la cellule-hôte ou bien du milieu de culture cellulaire.

7. Procédé pour la sécrétion par des cellules-hôtes procaryotiques de t-PA humain biologiquement actif qui subit une insolubilisation ou qui est toxique dans la cellule-hôte, les cellules-hôtes contenant un produit d'intégration d'ADN codant pour une ARN-polymérase exogène et étant transformées avec un acide nucléique codant pour une préprotéine du t-PA humain, dans lequel la préséquence effectue la sécrétion du t-PA dans la cellule-hôte, ledit acide nucléique étant sous le contrôle transcriptionnel d'un promoteur reconnu par l'ARN-polymérase exogène mais non reconnu par l'ARN-polymérase endogène de la cellule-hôte, l'expression de l'ARN-polymérase exogène étant sous le contrôle d'un promoteur inductible qui, à l'état non induit, engendre un degré d'expression suffisamment bas pour que la culture cellulaire soit stable dans cette condition au cours de générations successives, le procédé comprenant les étapes consistant à cultiver les transformants, à induire la transcription et à recueillir le t-PA humain mature à partir du cytoplasme cellulaire, de la membrane cellulaire ou bien du milieu de culture des cellules.
